# EUROPEAN PATENT SPECIFICATION

(11) **EP 4 039 839 B1**
(45) Date of publication and mention of the grant of the patent: **28.01.2026**
(21) Application number: 20871791.8
(22) Date of filing: 29.09.2020
(51) Int. Cl.: C22C 16/00, C22F 1/00, C22F 1/18, C22B 9/16, A61L 31/02, A61L 31/14, A61L 31/18, B22F 3/24, C22B 9/20, C22C 1/02, C22C 1/04

(54) **ZR-NB-BASED ALLOY MATERIAL, METHOD FOR MANUFACTURING SAID ALLOY MATERIAL, AND ZR-NB-BASED ALLOY PRODUCT**
LEGIERUNGSMATERIAL AUF ZR-NB-BASIS, VERFAHREN ZUR HERSTELLUNG DIESES LEGIERUNGSMATERIALS UND LEGIERUNGSPRODUKT AUF ZR-NB-BASIS
MATÉRIAU D'ALLIAGE À BASE DE ZR-NB, PROCÉDÉ DE FABRICATION DUDIT MATÉRIAU D'ALLIAGE ET PRODUIT D'ALLIAGE À BASE DE ZR-NB

(30) Priority: 03.10.2019 JP 2019182919
(43) Date of publication of application: 10.08.2022
(73) Proprietor: Proterial, Ltd., Tokyo 135-0061 (JP)
(72) Inventor: TAMURA, Shinya, Tokyo 100-8280 (JP); KIMURA, Tomonori, Tokyo 100-8280 (JP); AONO, Yasuhisa, Tokyo 100-8280 (JP)
(74) Representative: Strehl & Partner mbB
(86) International application number: PCT/JP2020/036836
(87) International publication number: WO 2021/065886

(56) References cited:
- WO-A1-2018/047611
- JP-A- 2010 075 413
- JP-A- 2010 075 413
- JP-A- 2020 084 300
- OKUNISHI E. ET AL: "HAADF-STEM studies of athermal and isothermal [omega]-phases in [beta]-Zr alloy", JOURNAL OF ALLOYS AND COMPOUNDS, vol. 577, 1 November 2013 (2013-11-01), CH, pages S713 - S716, XP093080804, ISSN: 0925-8388, DOI: 10.1016/j.jallcom.2011.12.115
- NOMURA NAOYUKI ET AL: "Effects of Phase Constitution of Zr-Nb Alloys on Their Magnetic Susceptibilities", vol. 50, no. 10, 1 October 2009 (2009-10-01), JP, pages 2466 - 2472, XP055815444, ISSN: 1345-9678, Retrieved from the Internet <URL:https://www.jstage.jst.go.jp/article/matertrans/50/10/50_M2009187/_pdf/-char/ja> DOI: 10.2320/matertrans.M2009187
- KONDO RYOTA ET AL: "Effect of Heat Treatment and the Fabrication Process on Mechanical Properties of Zr-14Nb Alloy", MATERIALS TRANSACTIONS, 1 January 2016 (2016-01-01), Sendai, pages 2060 - 2064, XP093080645, Retrieved from the Internet <URL:https://www.jstage.jst.go.jp/article/matertrans/57/12/57_MI201512/_pdf/-char/en> [retrieved on 20230911], DOI: 10.2320/matertrans.MI201512
- AKAHORI, TOSHIKAZU ET AL.: "Mechanical Properties and Biocompatibilities of Zr-Nb System Alloys with Different Nb Contents for Biomedical Applications", JOURNAL OF THE JAPAN INSTITUTE OF METALS AND MATERIALS, vol. 75, no. 8, 2011, pages 445 - 451, XP055815437
- NOMURA, NAOYUKI ET AL.: "Effects of Phase Constitution of Zr-Nb Alloys on Their Magnetic Susceptibilities", MATERIALS TRANSACTIONS, vol. 50, no. 10, 2009, pages 2466 - 2472, XP055815444

## Description

### Technical Field

The present invention relates to a low-magnetic susceptibility alloy and, more particularly, to a zirconium-niobium-based (Zr-Nb-based) alloy material, a method for manufacturing the alloy material, and a Zr-Nb-based alloy product.

### Background Art

Nowadays, medical examinations and surgery using magnetic resonance imaging (MRI) examinations are frequently conducted as a result of medical technological advancement. An MRI examination does not entail radiation exposure because radiation such as X-rays is not used. In addition, the examination is advantageous in that a high-resolution image can be obtained on any bodily cross section. However, because a high magnetic field is used, containing an internal instrument (implant) made of a high-magnetic susceptibility metal material may lead to the internal instrument being attracted to the magnetic field and moved or a virtual image or an obstructive shadow (referred to as an MRI artifact) being generated around the internal instrument. Accordingly, there is a weakness that no MRI examination can be performed on a person with an internal instrument that is made of a high-magnetic susceptibility metal material embedded in the body.

A problem that is unlikely to be detected by an X-ray examination (e.g. roentgenologic examination and computed tomography (CT) examination) can be detected by an MRI examination. Accordingly, the utilization of MRI examinations is expected to increase for more accurate diagnosis and treatment. Required from these viewpoints is a low-magnetic susceptibility alloy that can be suitably used as a biological metal material.

In addition, the internal instrument is required to be excellent in mechanical strength (e.g. hardness for wear resistance) from the viewpoint of long-term use.

Various alloys have been reported as low-magnetic susceptibility alloys for biological use. For example, JP-A-2006-265633 (PTL 1) discloses an MRI-compatible biological Co-Cr-Mo alloy that contains 28 to 35% by mass of chromium (Cr), 2 to 6% by mass of molybdenum (Mo), and a remainder being a composition of cobalt (Co) and unavoidable impurities.

According to PTL 1, it is possible to provide an MRI-compatible biological Co-Cr-Mo alloy that is excellent in mechanical properties such as wear resistance, has a magnetic susceptibility of 3 × 10⁻⁶ emu/g or less, and causes no MRI image distortion.

Disclosed in JP-A-2010-075413 (PTL 2) is a biological metal material that contains Zr as a main component. In this biological metal material, 0.5 to 15% by mass of at least one of titanium (Ti), vanadium (V), Cr, Nb, Mo, hafnium (Hf), tantalum (Ta), and tungsten (W) is contained as a subcomponent lower in content than the main component.

According to PTL 2, it is possible to provide a low-magnetic susceptibility biological metal material excellent in biocompatibility and mechanical properties and a medical device configured by such a metal material and capable of suppressing artifact generation in MRI diagnosis.

Disclosed in WO-A-2013/035269 (PTL 3) is a biological zirconium alloy that contains 27% by mol or more and 54% by mol or less of titanium, 5% by mol or more and 9% by mol or less of niobium as a β phase stabilization element stabilizing the β phase of zirconium, 1% by mol or more and 4% by mol or less in total amount of at least one of tin and aluminum as an ω phase suppression element suppressing the ω phase of zirconium, and the remainder consisting of zirconium and unavoidable impurities.

According to PTL 3, it is possible to provide a biological superelastic alloy that is even higher in workability and elasticity than existing Ti-nickel-based (Ti-Ni-based) and Ti-based high-elasticity alloys. In addition, since Zr, which has a small magnetic susceptibility, is contained in quantity, it is expected that the magnetic susceptibility of the alloy as a whole is small and MRI artifacts decrease.
The article OKUNISHI E. ET AL: "HAADF-STEM studies of athermal and isothermal ω-phases in β-Zr alloy", JOURNAL OF ALLOYS AND COMPOUNDS, vol. 577, 1 November 2013 (2013-11-01), pages S713-S716, relates to direct observations of athermal and isothermal ω-phases in β-Zr-alloy using dark-filed scanning transmission electron microscopy and draws conclusions on the orientation relationship between between the ω- and β- phases on the atomic scale.
The article NOMURA NAOYUKI ET AL: "Effects of Phase Constitution of Zr-Nb Alloys on Their Magnetic Susceptibilities", MATERIALS TRANSACTIONS, vol. 50, no. 10, 1 October 2009 (2009-10-01), pages 2466-2472 studies the magnetic susceptibility of such materials.
The article Kondo Ryota ET AL: "Effect of Heat Treatment and the Fabrication Process on Mechanical Properties of Zr-14Nb Alloy", MATERIALS TRANSACTIONS, 1 January 2016 (2016-01-01), pages 2060-2064, investigates the effects of thermomechanical processing of such materials on the microstructure and mechanical properties thereof.

### Citation List

### Patent Literature

PTL 1: JP-A-2006-265633
PTL 2: JP-A-2010-075413
PTL 3: WO-A-2013/035269

### Summary of Invention

### Technical Problem

As described above, a metal material (biological metal material) configuring a biological device requires a low-magnetic susceptibility alloy from the viewpoint of usability in MRI examinations. In addition, excellent mechanical strength is required from the viewpoint of long-term use. Indispensable for use as a biological metal material is suppression of metal ion elution attributable to in-vivo corrosion (suppression of cytotoxicity attributable to eluted metal ions). In other words, the balance between low magnetic susceptibility, mechanical strength, and corrosion resistance is important for biological metal materials.

It can be said that the biological alloys described in PTL 1, PTL 2, and PTL 3 have achieved a low magnetic susceptibility lower than the magnetic susceptibility of Ti and a satisfactory mechanical strength. However, the biological alloys described in PTL 1, PTL 2, and PTL 3 lack corrosion resistance-related experiments and demonstrations and the balance between low magnetic susceptibility, mechanical strength, and corrosion resistance is yet to be confirmed.

In this regard, an object of the present invention is to provide a Zr-Nb-based alloy material excellent in balance between low magnetic susceptibility, mechanical strength, and corrosion resistance, a method for manufacturing the alloy material, and a Zr-Nb-based alloy product.

### Solution to Problem

(I) In order to solve the above problem, the invention proposes a Zr-Nb-based alloy material as specified in claim 1, a method for manufacturing the Zr-Nb-based alloy material according to claim 5 and a Zr-Nb-based alloy product according to claim 10.
   The present invention can be improved or modified as follows in the above Zr-Nb-based alloy material (I) according to the present invention.
   (i) The isothermal ω phase particles have a chemical composition in which the Zr content is high and the Nb content is low as compared with the β phase crystal grains.
   (ii) The isothermal ω phase particles have an average particle size of 200 nm or less.
   (iii) The Zr-Nb-based alloy material is 2.0 × 10⁻⁶ cm³/g or less in mass magnetic susceptibility σ, 300 HV or more in Vickers hardness HV, and 750 mV (vs. Ag/AgCl) or more in pitting potential V_{C100}.
   It should be noted that those satisfying this characteristic requirement are regarded in the present invention as balanced in terms of low magnetic susceptibility, mechanical strength, and corrosion resistance.
(II) In another aspect of the present invention, a method for manufacturing the Zr-Nb-based alloy material includes: a raw material mixing and melting step of forming a molten metal by mixing and melting raw materials of the Zr-Nb-based alloy material; a casting step of forming a cast material having the chemical composition by casting the molten metal; a solution treatment step of preparing a solution-treated material by performing solution treatment at 950°C or more and 1,100°C or less on the cast material; and an aging treatment step of obtaining an aging-treated material by performing aging treatment at 100°C or more and 450°C or less on the solution-treated material.
   The present invention can be improved or modified as follows in the above method for manufacturing the Zr-Nb-based alloy material (II) according to the present invention.
   (v) The method for manufacturing the Zr-Nb-based alloy material further includes a plastic working step of forming a plastically worked material by performing plastic working on the solution-treated material between the solution treatment step and the aging treatment step, in which the aging treatment step is performed on the plastically worked material.
   (vi) The method for manufacturing the Zr-Nb-based alloy material further includes a plastic working step of forming a plastically worked material by performing plastic working on the cast material between the casting step and the solution treatment step, in which the solution treatment step is performed on the plastically worked material.
   (vii) The method for manufacturing the Zr-Nb-based alloy material further includes a machining step of forming a machined material by machining the aging-treated material after the aging treatment step.
   (viii) The raw material mixing and melting step includes: a raw material alloy ingot forming element step of forming a raw material alloy ingot by temporarily solidifying the molten metal after forming the molten metal by mixing and melting the raw materials; and a remelting element step of preparing a purified molten metal by remelting the raw material alloy ingot.
(III) In still another aspect of the present invention, a method for manufacturing the Zr-Nb-based alloy material includes: a powder preparation step of preparing additive manufacturing powder adjusted so as to have the chemical composition; an additive manufacturing step of forming an additively manufactured product having a desired three-dimensional shape using the additive manufacturing powder; a solution treatment step of preparing a solution-treated material by performing solution treatment at 950°C or more and 1,100°C or less on the additively manufactured product; and an aging treatment step of obtaining an aging-treated material by performing aging treatment at 100°C or more and 450°C or less on the solution-treated material.
(IV) In yet another aspect of the present invention, a Zr-Nb-based alloy product using a Zr-Nb-based alloy material, in which the Zr-Nb-based alloy material is the Zr-Nb-based alloy material described above and the product is a stent or a spring guide wire.

### Advantageous Effects of Invention

According to the present invention, it is possible to provide a Zr-Nb-based alloy material excellent in balance between low magnetic susceptibility, mechanical strength, and corrosion resistance, a method for manufacturing the alloy material, and a Zr-Nb-based alloy product.

### Brief Description of Drawings

[Fig. 1] Fig. 1 is a step diagram illustrating an example of a method for manufacturing a Zr-Nb-based alloy material according to the present invention.
[Fig. 2] Fig. 2 is a step diagram illustrating another example of the method for manufacturing the Zr-Nb-based alloy material according to the present invention.
[Fig. 3] Fig. 3 is a step diagram illustrating still another example of the method for manufacturing the Zr-Nb-based alloy material according to the present invention.
[Fig. 4A] Fig. 4A is an enlarged schematic diagram illustrating a part of a stent as an example of a product using a Zr-Nb-based alloy material of the present invention.
[Fig. 4B] Fig. 4B is a schematic perspective diagram of a spring guide wire as another example of the product using the Zr-Nb-based alloy material of the present invention.
[Fig. 5] Fig. 5 is a TEM observation image of an aging-treated material A-3 as the alloy material of the present invention, in which Fig. 5(a) is a dark field image and Fig. 5(b) is an electron beam diffraction image.

### Description of Embodiments

The present inventors have completed the present invention by diligently investigating and studying chemical composition, metal structure morphology, mass magnetic susceptibility, mechanical strength, and corrosion resistance relationships with regard to a Zr-Nb-based alloy material containing Zr and Nb as essential components, a Zr-Nb-based alloy material containing 65% by mass or more of Zr in particular.

Hereinafter, an embodiment of the present invention will be specifically described with reference to the drawings. The present invention is not limited to the embodiment. The present invention can be appropriately combined with or improved based on a known technique within the technical idea of the invention. It should be noted that the same reference numerals are given to synonymous states and steps with redundant description omitted.

### [Chemical Composition of Zr-Nb-based Alloy Material]

First, the chemical composition of the Zr-Nb-based alloy material of the present invention will be described.

### Nb: 3% by mass or more and 18% by mass or less

Nb, which is an essential component of the Zr-Nb-based alloy material of the present invention, stabilizes the β phase and contributes to ensuring mechanical strength in this alloy material. The mechanical strength of the Zr-Nb-based alloy material is sufficient when the Nb content is 3% by mass or more. In order to obtain the action and effect of β phase stabilization and mechanical strength, the Nb content is preferably 3% by mass or more, more preferably 5% by mass or more, and even more preferably 8% by mass or more. On the other hand, when the Nb content is 18% by mass or less, it is difficult to inhibit magnetic susceptibility reduction by Zr. The upper limit of the Nb content is preferably 18% by mass or less, more preferably 16% by mass or less, and even more preferably 15% by mass or less. It should be noted that the upper and lower limit values of the Nb content can be combined in any manner. The same applies to the following elements.

### Ti: 3% by mass or more and 12% by mass or less

Ti is one of the components of the Zr-Nb-based alloy material of the present invention (is not an essential component of the alloy material and may or may not be added). Ti contributes to the stabilization of the β phase together with Nb. In addition, Ti contributes to corrosion resistance improvement. In order to obtain the action and effect resulting from Ti addition, 3% by mass or more is needed, 4% by mass or more is preferable, and 5% by mass or more is even more preferable. When the Ti content is less than 3% by mass, the above action and effect tend to be insufficient. On the other hand, when the Ti content exceeds 12% by mass, magnetic susceptibility reduction by Zr tends to be inhibited. The upper limit of the Ti content is preferably 12% by mass or less, more preferably 11% by mass or less, and even more preferably 10% by mass or less.

### Cr: 0.5% by mass or more 6% by mass or less

Cr is also one of the components of the Zr-Nb-based alloy material of the present invention. Cr contributes to the stabilization of the β phase and corrosion resistance improvement. In addition, Cr contributes more to the stabilization of the β phase by being added together with Ti. In order to obtain the action and effect resulting from Cr addition, 0.5% by mass or more is needed, 0.8% by mass or more is preferable, and 1% by mass or more is even more preferable. When the Cr content is less than 0.5% by mass, the above action and effect tend to be insufficient. On the other hand, when the Cr content exceeds 6% by mass, a coarse intermetallic compound (e.g. ZrCr₂) is formed to result in a decline in mechanical strength and magnetic susceptibility reduction by Zr tends to be inhibited. The upper limit of the Cr content is preferably 6% by mass or less, more preferably 5% by mass or less, and even more preferably 4% by mass or less.

### Cu: 6% by mass or less

Cu is one of the optional components of the Zr-Nb-based alloy material of the present invention. Cu contributes to magnetic susceptibility reduction. In order to obtain the action and effect resulting from Cu addition, 0.5% by mass or more is preferable, 0.8% by mass or more is more preferable, and 1% by mass or more is even more preferable. When the Cu content is less than 0.5% by mass, the above action and effect are insufficient and yet there is no particular problem. On the other hand, when the Cu content exceeds 6% by mass, a coarse intermetallic compound is formed and a decline in corrosion resistance tends to arise. The upper limit of the Cu content is preferably 6% by mass or less, more preferably 3% by mass or less, and even more preferably 2.5% by mass or less.

### Bi: 5% by mass or less

Bi is also one of the optional components of the Zr-Nb-based alloy material of the present invention. Bi contributes to magnetic susceptibility reduction. In order to obtain the action and effect resulting from Bi addition, 0.5% by mass or more is preferable, 0.8% by mass or more is more preferable, and 1% by mass or more is even more preferable. When the Bi content is less than 0.5% by mass, the above action and effect are insufficient and yet there is no particular problem. On the other hand, when the Bi content exceeds 5% by mass, a coarse intermetallic compound is formed and a decline in corrosion resistance tends to arise. The upper limit of the Bi content is preferably 5% by mass or less, more preferably 3% by mass or less, and even more preferably 2.5% by mass or less.

### Remainder: Zr and unavoidable impurities

Zr is the main component (component of the maximum content) of the Zr-Nb-based alloy material of the present invention and contributes to lowering the magnetic susceptibility of this alloy material. In order to obtain the action and effect of lowering the magnetic susceptibility, the Zr content is preferably 65% by mass or more, more preferably 70% by mass or more, and even more preferably 75% by mass or more. When the Zr content is less than 65% by mass, lowering the magnetic susceptibility of the Zr-Nb-based alloy material tends to be insufficient. On the other hand, when the Zr content exceeds 94% by mass, the mechanical strength (e.g. Vickers hardness) of the Zr-Nb-based alloy material tends to be insufficient. The upper limit of the Zr content is preferably 94% by mass or less, more preferably 90% by mass or less, and even more preferably 85% by mass or less.

Examples of the unavoidable impurities in the Zr-Nb-based alloy material of the present invention include Hf, oxygen (O), carbon (C), nitrogen (N), phosphorus (P), and sulfur (S). Mechanical properties and corrosion resistance deteriorate when these impurities are contained excessively. Accordingly, the total content is 0.5% by mass or less, Hf is less than 0.5% by mass, O, C, and N are 0.5% by mass or less each, and P and S are 0.1% by mass or less each.

The Zr-Nb-based alloy material of the present invention is capable of further containing the following elements as optional components.

### Ag: 9% by mass or less

Ag is also one of the optional components of the Zr-Nb-based alloy material of the present invention and contributes to magnetic susceptibility reduction. In a case where Ag is added, 0.5% by mass or more is preferable, 0.8% by mass or more is more preferable, and 1% by mass or more is even more preferable. When the Ag content is less than 0.5% by mass, the above action and effect may not be sufficient. On the other hand, when the Ag content exceeds 9% by mass, a coarse intermetallic compound may be formed and a decline in corrosion resistance may arise. The upper limit of the Ag content is preferably 9% by mass or less, more preferably 5% by mass or less, and even more preferably 2.5% by mass or less.

### Sn: 6% by mass or less

Sn is also one of the optional components of the Zr-Nb-based alloy material of the present invention and contributes to magnetic susceptibility reduction. In a case where Sn is added, 0.5% by mass or more is preferable, 1% by mass or more is more preferable, and 1.5% by mass or more is even more preferable. When the Sn content is less than 0.5% by mass, the above action and effect may not be sufficient. On the other hand, when the Sn content exceeds 6% by mass, a coarse intermetallic compound (e.g. ZrSn) may be formed and a decline in corrosion resistance may arise. The upper limit of the Sn content is preferably 6% by mass or less, more preferably 5% by mass or less, and even more preferably 4% by mass or less.

### Al: 3% by mass or less

Al is also one of the optional components of the Zr-Nb-based alloy material of the present invention and contributes to magnetic susceptibility reduction. In a case where Al is added, 0.5% by mass or more is preferable, 0.8% by mass or more is more preferable, and 1% by mass or more is even more preferable. When the Al content is less than 0.5% by mass, the above action and effect may not be sufficient. On the other hand, when the Al content exceeds 3.0% by mass, a coarse intermetallic compound (e.g. ZrAl₃) may be formed and a decline in corrosion resistance may arise. The upper limit of the Al content is preferably 3% by mass or less, more preferably 2.8% by mass or less, and even more preferably 2.5% by mass or less.

### [Microstructure of Zr-Nb-based Alloy Material]

The microstructure (also referred to as metallographic structure) of the Zr-Nb-based alloy material of the present invention will be described.

α, β, and ω phases can be mentioned as the main constituent phases in the Zr-Nb-based alloy. The α phase has a hexagonal close-packed structure and is relatively low in mechanical strength. In addition, the α phase has a small solid solution limit for another element. Accordingly, in terms of properties, an intermetallic compound is easily formed and the corrosion resistance is relatively low when another element is added. The β phase has a body-centered cubic lattice structure and exhibits relatively high mechanical strength and satisfactory corrosion resistance. The ω phase is generated in the process of the phase transformation from β phase to α phase and has the structure of a hexagonal crystal system. In addition, the ω phase has an advantage that the magnetic susceptibility is small from the relationship of atomic arrangement and yet has a weakness that the corrosion resistance is relatively low.

It should be noted that the ω phase includes an athermal ω phase formed in a cooling process (quenching) such as a casting step and an isothermal ω phase formed in an isothermal process such as aging treatment. The athermal ω phase is generated as a result of shear deformation, which is a reversible transformation. Accordingly, the athermal ω phase is likely to be formed between β phase crystal grains and is identical in composition to the β phase before the phase transformation. On the other hand, the isothermal ω phase is generated and grows as a result of atomic diffusion (atomic rearrangement), which is an irreversible transformation. Accordingly, the isothermal ω phase is likely to be formed in β phase crystal grains and is different in composition from the β phase before the phase transformation. In other words, the athermal and isothermal ω phases are clearly different from each other in terms of the process of generation, morphology, and composition.

The metallographic structure of the Zr-Nb-based alloy material of the present invention is characterized in that fine isothermal ω phase particles are dispersed and precipitated in β phase crystal grains as a parent phase (matrix). The advantages of the β phase (relatively high mechanical strength and satisfactory corrosion resistance) can be enjoyed by the β phase crystal grains being used as the parent phase. In addition, since the fine isothermal ω phase particles are dispersed and precipitated in the β phase crystal grains, the advantage of the ω phase (magnetic susceptibility reduction) can be enjoyed and a contribution to mechanical strength improvement (e.g. dispersion and precipitation enhancement) is made. Further, since the ω phase particles are not precipitated at the grain boundaries of the β phase crystal grains, the weakness of corrosion resistance can be minimized.

Resultantly obtained according to the present invention are, for example, a mass magnetic susceptibility (σ) of 2.0 × 10⁻⁶ cm³/g or less, a Vickers hardness (HV) of 300 HV or more, and a pitting potential (V_{C100}) of 750 mV (vs. Ag/AgCl) or more in a case where the reference electrode is a silver/silver chloride electrode. As for the pitting potential V_{C100}, the potential at the time of a current density of 100 µA/cm² being reached after the initiation of a sudden current rise attributable to the occurrence of pitting corrosion in an anode polarization curve was measured.

From the viewpoint of magnetic susceptibility reduction, the mass magnetic susceptibility σ is more preferably 1.6 × 10⁻⁶ cm³/g or less and even more preferably 1.3 × 10⁻⁶ cm³/g or less. In addition, from the viewpoint of mechanical strength, the Vickers hardness HV is more preferably 350 or more and even more preferably 400 or more. Further, from the viewpoint of corrosion resistance, the pitting potential V_{C100} is more preferably 1,000 mV or more and even more preferably 1,250 mV or more.

Although the size of the isothermal ω phase particles is not particularly limited, the average particle size is preferably 200 nm or less, more preferably 150 nm or less, and even more preferably 100 nm or less from the viewpoint of dispersion and precipitation. The average particle size exceeding 200 nm leads to deterioration of mechanical properties attributable to precipitate coarsening and the progress of α transformation of the ω phase entailed by grain growth, which may result in deterioration of corrosion resistance and magnetic susceptibility characteristics. From the viewpoint of magnetic susceptibility reduction, an average particle size of 10 nm or more is preferable. When the average particle size is less than 10 nm, it is difficult to ensure the amount of ω phase precipitation that is effective in reducing magnetization.

Although it is desirable that a heterogeneous phase other than the β and ω phases (e.g. intermetallic compound or α phase) is not detected in the Zr-Nb-based alloy material of the present invention, the heterogeneous phase is acceptable insofar as the heterogeneous phase has no particular adverse effect on corrosion resistance and mechanical properties (e.g. the total occupancy of the heterogeneous phase being 10% or less).

In the present invention, an average particle size and an area occupancy (area%) analyzed and calculated using an existing image processing technology with respect to a microstructure observation image are adopted regarding the average particle size of the isothermal ω phase particles and the occupancy of the heterogeneous phase. For example, it is possible to calculate the area ratio of the heterogeneous phase as an object of analysis and the average particle size of the isothermal ω phase particles (average of the diameters of equivalent area circles) by reading an optical or electron microscope observation image of a cross section of the alloy material with image analysis software (e.g. ImageJ, public domain software developed by the U.S. National Institutes of Health).

### [Zr-Nb-based Alloy Material Manufacturing Method - 1]

Next, a method for manufacturing the Zr-Nb-based alloy material of the present invention will be described.

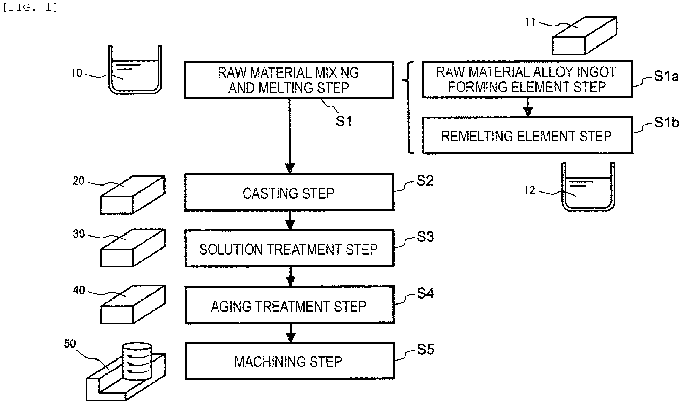

Fig. 1 is a step diagram illustrating an example of the method for manufacturing the Zr-Nb-based alloy material according to the present invention. Performed first as illustrated in Fig. 1 is a raw material mixing and melting step (S1) of forming a molten metal 10 by mixing and melting the raw materials of the Zr-Nb-based alloy material such that a desired composition (essential and optional components) is obtained. The method by which the raw materials are mixed and the method by which the raw materials are melted are not particularly limited, and existing alloy material manufacturing methods can be used.

In addition, for a further decline in the impurity component content of the alloy material (for alloy cleanliness enhancement), it is more preferable that the raw material mixing and melting step S1 includes a raw material alloy ingot forming element step (S1a) of forming a raw material alloy ingot 11 by temporarily solidifying the molten metal 10 after forming the molten metal 10 by mixing and melting the raw materials of the Zr-Nb-based alloy material and a remelting element step (S1b) of preparing a purified molten metal 12 by remelting the raw material alloy ingot 11. The method by which the remelting is performed is not particularly limited insofar as the alloy cleanliness can be enhanced, but vacuum arc remelting (VAR) can be preferably used.

Performed next is a casting step (S2) of forming a cast material 20 by casting the molten metal 10 using a predetermined mold. In a case where the remelting element step S1b is performed as described above, the casting step S2 is a step of forming the cast material 20 by casting the purified molten metal 12. In the case of precision casting (in a case where a cast material with a shape close to the shape of the final product is to be obtained), a large mother alloy ingot may be fabricated by temporarily casting the molten metal 10 component-adjusted in the raw material mixing and melting step S1, the mother alloy ingot may be divided into parts appropriate in size, and then remelting and casting with a precision casting mold may be performed. In that case, it is preferable from the viewpoint of the mechanical properties and corrosion resistance of the final product to ensure a cooling rate at which crystal grain coarsening during solidification (coarse casting solidification structure) can be suppressed.

Performed next is a solution treatment step (S3) of preparing a solution-treated material 30 by performing solution heat treatment at 950°C or more and 1,100°C or less on the cast material 20. The progress of β phase formation occurs at 950°C or more. At 1,100°C or less, it is possible to suppress the progress of component oxidation and the occurrence of component unevenness. As for the retention time in the heat treatment, the time length may be appropriately adjusted with the retention temperature and the heat capacity of the material to be heat-treated taken into account (e.g. 0.1 hours or more and 20 hours or less). By performing the solution heat treatment, the heterogeneous phase generated in the cooling process of the casting step S2 (e.g. α phase, athermal ω phase, and intermetallic compound phase) goes through solutionizing (phase transformation and/or solidification) into the β phase and monophase conversion into the β phase proceeds. As the microstructure, a structure in which recrystallized grains can be seen (recrystallized structure) may be shown by performing the solution treatment step S3.

Methods for cooling from the solution temperature in the solution heat treatment are not particularly limited. A cooling method by which a high cooling rate can be ensured (e.g. water cooling and gas cooling) is preferably usable from the viewpoint of maintaining the microstructure as much as possible.

Performed next is an aging treatment step (S4) of preparing an aging-treated material 40 by performing aging heat treatment at 100°C or more and 450°C or less on the solution-treated material 30. At 100°C or more, component diffusion occurs substantially and the effect of aging can be obtained. In addition, an ω phase transformation structure is formed at 450°C or less. As for the retention time in the heat treatment, the time length may be appropriately adjusted with the retention temperature and the heat capacity of the material to be heat-treated taken into account (e.g. 0.1 hours or more and 50 hours or less). Obtainable by performing the aging heat treatment is the aging-treated material 40 having a microstructure in which fine isothermal ω phase particles are dispersed and precipitated in the β phase crystal grains of the solution-treated material 30.

Performed next is a machining step (S5) of forming a machined material 50 by machining the aging-treated material 40 such that a desired shape is given. The machining in the present invention means machining performed using a machine tool for molding into a desired shape (e.g. cutting, grinding, electric discharge machining, laser machining, and water jet machining). The machining step S5 is inessential, and the aging-treated material 40 may be used as a finished product of the Zr-Nb-based alloy material.

If necessary, a finishing step (not illustrated) of completing the Zr-Nb-based alloy material by performing finishing (e.g. surface polishing) on the machined material 50 may be performed after the machining step S5. The finishing step is also inessential, and the machined material 50 may be used as a finished product of the Zr-Nb-based alloy material.

### [Zr-Nb-based Alloy Material Manufacturing Method - 2]

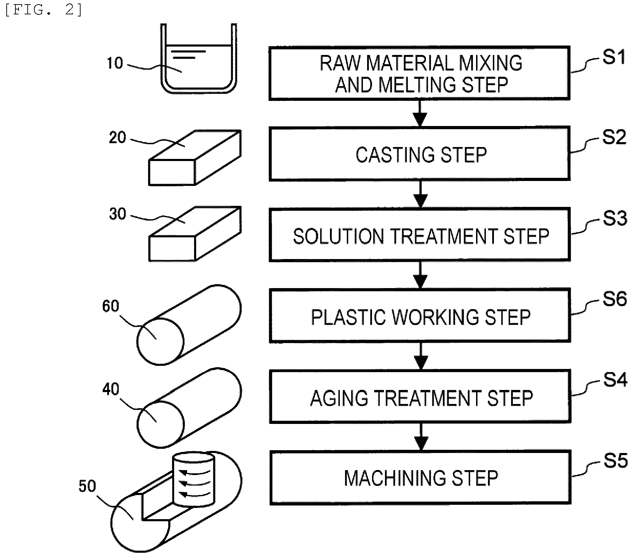

Fig. 2 is a step diagram illustrating another example of the method for manufacturing the Zr-Nb-based alloy material according to the present invention. Although the illustration of the raw material alloy ingot forming element step S1a and the remelting element step S1b in Fig. 1 is omitted for the purpose of simplification of the drawings, it is a matter of course that the element steps may be performed.

Fig. 2 is different in that a plastic working step (S6) is provided between the solution treatment step S3 and the aging treatment step S4 in the manufacturing method of Fig. 1, and the other steps are the same. Accordingly, only the plastic working step S6 will be described below.

In the manufacturing method illustrated in Fig. 2, the plastic working step S6 of forming a plastically worked material 60 by performing plastic working on the solution-treated material 30 obtained in the solution treatment step S3 is performed. The plastic working is not particularly limited in terms of type and method, and existing types and methods (e.g. forging, extrusion, drawing, and rolling including hot, warm, and cold forging, extrusion, drawing, and rolling) can be used.

As a result of the plastic working, the casting solidification structure caused by the casting step S2 and/or the recrystallized structure caused by the solution treatment step S3 is broken, a microstructure smaller in average crystal grain size than the crystal grains of the solution-treated material 30 is formed, and the plastically worked material 60 of the Zr-Nb-based alloy that has a brittleness suppression effect can be obtained. If necessary, a refining heat treatment (not illustrated in Fig. 2) for adjusting the hardness of the plastically worked material 60 may be performed after the plastic working.

Subsequently, the same aging treatment step S4, machining step S5, and finishing step as in the manufacturing method of Fig. 1 may be performed except that the aging-treated material 40 is prepared by performing the aging treatment step S4 on the plastically worked material 60.

### [Zr-Nb-based Alloy Material Manufacturing Method - 3]

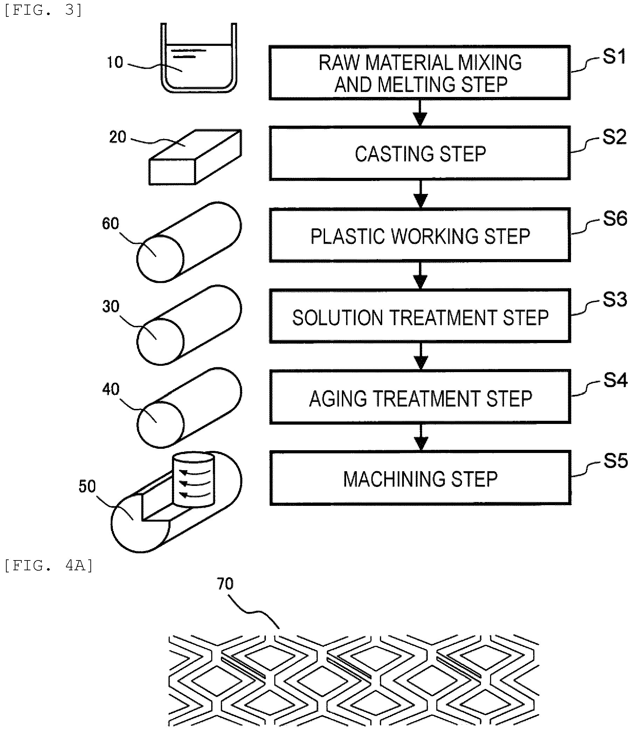

Fig. 3 is a step diagram illustrating still another example of the method for manufacturing the Zr-Nb-based alloy material according to the present invention. Although the illustration of the raw material alloy ingot forming element step S1a and the remelting element step S1b in Fig. 1 is omitted for the purpose of simplification of the drawings, it is a matter of course that the element steps may be performed.

Fig. 3 is different in that the plastic working step S6 is provided between the casting step S2 and the solution treatment step S3 in the manufacturing method of Fig. 1, and the other steps are the same. In addition, the type and method of the plastic working in the plastic working step S6 are the same as in Fig. 2. In other words, only the order in which the plastic working step S6 is performed is different as compared with the manufacturing method of Fig. 2.

By performing the plastic working on the cast material 20, the casting solidification structure caused by the casting step S2 is broken, a microstructure smaller in average crystal grain size than the crystal grains of the cast material 20 is formed, and the plastically worked material 60 of the Zr-Nb-based alloy that has a brittleness suppression effect can be obtained. In addition, the crystal grain size of the heterogeneous phase in the cast material 20 (e.g. α phase, athermal ω phase, and intermetallic compound phase) also decreases, and thus the advantage also includes promoting the monophase conversion of the β phase in the following solution treatment step S3.

Subsequently, the same solution treatment step S3, aging treatment step S4, machining step S5, and finishing step as in the manufacturing method of Fig. 1 may be performed except that the solution-treated material 30 is prepared by performing the solution treatment step S3 on the plastically worked material 60.

### [Zr-Nb-based Alloy Material Manufacturing Method - 4]

An additive manufacturing method is applicable as yet another example of the method for manufacturing the Zr-Nb-based alloy material according to the present invention. For example, a step of preparing raw material alloy powder having a desired composition or mixed powder adjusted so as to have a desired composition as additive manufacturing powder and an additive manufacturing step of forming an additively manufactured product having a desired three-dimensional shape using the additive manufacturing powder are performed instead of the S1 and S2 steps in Fig. 3. Subsequently, the solution treatment step S3 and the aging treatment step S4 are performed on the additively manufactured product. By this manufacturing method, a target member having a three-dimensional shape can be manufactured regardless of plastic working.

If necessary, the machining step S5 may be performed before and after the heat treatment step. Performing heat treatment after target member shape machining is also advantageous in that the structure does not deteriorate due to the shape machining.

### [Product Examples Using Zr-Nb-based Alloy Material]

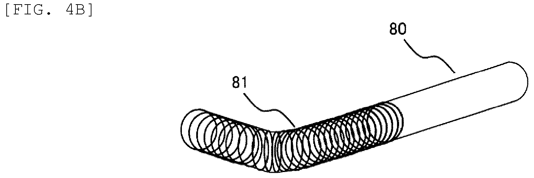

Next, product examples using the Zr-Nb-based alloy material of the present invention will be described briefly. Fig. 4A is an enlarged schematic diagram illustrating a part of a stent as an example of a product, and Fig. 4B is a perspective schematic diagram of a spring guide wire as an example of another product.

As illustrated in Figs. 4A and 4B, the Zr-Nb-based alloy material of the present invention can be suitably used as a constituent material of a stent 70 or a core 81 of a spring guide wire 80 since the magnetic susceptibility of the Zr-Nb-based alloy material of the present invention is equivalent to or less than the magnetic susceptibility of the biological alloy of the related art and the Zr-Nb-based alloy material of the present invention has high mechanical strength and corrosion resistance.

### [Examples]

Hereinafter, the present invention will be described in more detail with reference to examples and comparative examples. It should be noted that the present invention is not limited to the examples.

### (Fabrication of Cast Materials C-1 to C-7)

Raw materials were mixed and melted by an arc melting method (at a melting temperature of 1500°C or more in a reduced-pressure Ar atmosphere) for the nominal chemical compositions in Table 1 below. After the resultant molten metal formation (after the raw material mixing and melting step S1), cast materials C-1 to C-7 were fabricated by solidification on a water-cooled copper hearth (casting step S2). Test pieces were respectively collected from the obtained cast materials C-1 to C-7. C-1 is not according to the present invention as defined in the claims.

**[Table 1]**

| Table 1: Nominal chemical compositions of cast materials C-1 to C-7 | | | | | | |
|---|---|---|---|---|---|---|
| Cast material | Nominal chemical composition (% by mass) | | | | | |
| | Zr | Nb | Ti | Cr | Cu | Bi |
| C-1 | Bal. | 10 | - | - | - | - |
| C-2 | Bal. | 15 | 10 | 3 | - | - |
| C-3 | Bal. | 15 | 5 | 3 | - | - |
| C-4 | Bal. | 15 | 3 | 3 | - | - |
| C-5 | Bal. | 15 | 10 | 5 | - | - |
| C-6 | Bal. | 15 | 10 | 3 | 1 | - |
| C-7 | Bal. | 15 | 10 | 3 | - | 2.5 |

| | | | | | | |
|---|---|---|---|---|---|---|
| "-" in the table means that each was not intentionally included. | | | | | | |

In Table 1, the content of each component (unit: % by mass) is converted such that the total of the described components is 100% by mass. In addition, impurities other than the elements listed in Table 1 (Hf, O, C, N, P, and S) have a total content of 0.5% by mass or less and are included in "Bal.".

The cast material C-1 is a reference example of a Zr-Nb-based alloy consisting only of Zr and Nb. The cast materials C-2 to C-5 are examples of a Zr-Nb-based alloy containing Ti and Cr in addition to Zr and Nb. The cast material C-6 is an example of a Zr-Nb-based alloy containing Ti, Cr, and Cu in addition to Zr and Nb. The cast material C-7 is an example of a Zr-Nb-based alloy containing Ti, Cr, and Bi in addition to Zr and Nb.

### (Fabrication of Solution-treated Materials S-1 to S-7)

Solution heat treatment (water cooling after holding for 30 minutes at 1,000°C) was performed on the cast materials C-1 to C-7 prepared in the casting step S2. As a result, solution-treated materials S-1 to S-7 were fabricated (solution treatment step S3). Test pieces were respectively collected from the obtained solution-treated materials S-1 to S-7.

### (Fabrication of Aging-treated Materials A-1 to A-7)

Next, aging heat treatment (air cooling after holding for 30 minutes at 400°C) was performed on the solution-treated materials S-1 to S-7 prepared in the solution treatment step S3. As a result, aging-treated materials A-1 to A-7 were fabricated (aging treatment step S4). Test pieces were respectively collected from the obtained aging-treated materials A-1 to A-7. The aging-treated materials A-1 to A-7 are the Zr-Nb-based alloy materials according to the present invention.

### (Testing and Evaluation of Cast, Solution-treated, and Aging-treated Materials)

### (1) Microstructure Observation

Mirror polishing was performed on the surfaces of the collected test pieces of the cast materials C-1 to C-7, the solution-treated materials S-1 to S-7, and the aging-treated materials A-1 to A-7. Then, X-ray diffraction measurement (acceleration voltage 48 kV, tube current 28 mA) was performed to investigate the crystal phase by which each test piece is configured. The results are as shown in Table 2.

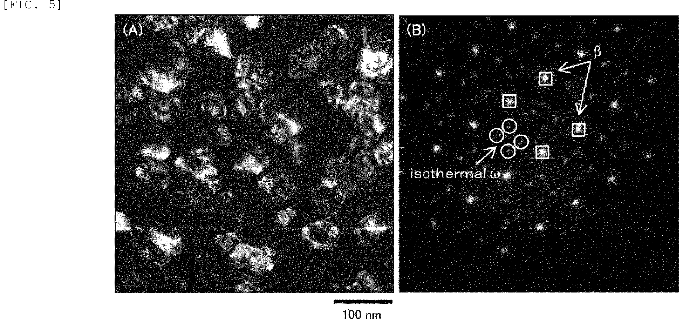

In addition, microstructure observation (acceleration voltage 200 kV, magnification × 40,000) was performed on the test pieces of the aging-treated materials A-1 to A-7 using a transmission electron microscope-energy dispersive X-ray analyzer (TEM-EDX, Hitachi High-Technologies Corporation, model HF-2100 Cold-FE-TEM). In this manner, the average particle size, crystal structure, and chemical composition of the isothermal ω phase particles were investigated. The results are as shown in Fig. 5 and Table 3.

**[Table 2]**

| Table 2: Result of detection phase by X-ray diffraction measurement of cast material, solution-treated material, and aging-treated material | |
|---|---|
| | Detection phase |
| Cast materials C-1 to C-7 | α phase + β phase + ω phase + intermetallic compound phase |
| Solution-treated materials S-1 to S-7 | β phase |
| Aging-treated materials A-1 to A-7 | β phase |

As for the cast materials C-1 to C-7, "α phase + β phase + ω phase + intermetallic compound phase" was detected as a result of X-ray diffraction measurement as shown in Table 2. On the other hand, as for the solution-treated materials S-1 to S-7, it is confirmed that only the β phase was detected and the α, ω, and intermetallic compound phases went through solidification and monophase conversion into the β phase as a result of solution heat treatment.

As for the aging-treated materials A-1 to A-7, the ω phase was not detected as a result of X-ray diffraction measurement contrary to expectations. This suggests that the isothermal ω phase particles precipitated by the aging-treated materials A-1 to A-7 are very fine. In other words, this means that athermal ω phase particles having a size detectable by X-ray diffraction measurement are precipitated in the cast materials C-1 to C-7.

Fig. 5 is a TEM observation image of the aging-treated material A-3, which is an alloy material of the present invention. Fig. 5(a) is a dark field image, and Fig. 5(b) is an electron beam diffraction image. As shown in the dark field image of Fig. 5(a), it is confirmed that fine particles with an average particle size of approximately 60 nm are dispersed and precipitated in the parent phase crystal (β phase). Confirmed from the electron beam diffraction image of Fig. 5(b) is that the ω phase having a hexagonal crystal structure different from the β phase of the body-centered cubic lattice structure is precipitated. It was also confirmed that the ω phase with an average particle size of 200 nm or less is finely precipitated in the aging-treated materials other than A-3 as well.

**[Table 3]**

| Table 3: Result of quantitative analysis of β phase parent phase and ω phase particles by TEM-EDX of aging-treated material A-3 | | | |
|---|---|---|---|
| Crystal phase | Chemical composition (atomic%) | | |
| | Zr | Nb | Cr |
| β phase | 76.0 | 11.2 | 2.8 |
| ω phase | 85.5 | 6.2 | 0.9 |

As shown in Table 3, it is confirmed in terms of chemical composition that the ω phase particles are higher in Zr content and lower in Nb content and Cr content than the β phase parent phase. In this microstructure observation, a Ti holder was used as a TEM-EDX measurement sample holder and thus Ti measurement is excluded from the quantitative analysis of the chemical composition.

Considering the results in Table 3 and Fig. 5 together, it is confirmed that the finely dispersed and precipitated ω phase is an isothermal ω phase. In addition, it was separately confirmed that the same results as in Fig. 5 and Table 3 can be obtained from the other aging-treated materials (A-1 to A-2 and A-4 to A-7).

Next, the test pieces of the solution-treated materials S-1 to S-7 and the aging-treated materials A-1 to A-7 were evaluated for magnetization characteristics, mechanical strength, and corrosion resistance. The aging-treated materials A-1 to A-7 are examples of the present invention, and the solution-treated materials S-1 to S-7 are comparative examples.

### (2) Evaluation of Magnetization Characteristics

As an evaluation of magnetization characteristics, magnetization measurement was performed using a magnetic material measuring device (RIKEN Electronics Co., Ltd., vibration sample-type magnetometer BHV series). The mass magnetic susceptibility β (× 10⁻⁶ cm³/g) was calculated from the relationship between the obtained magnetization, applied magnetic field, and sample mass.

As a result of the magnetization measurement, "2.00 < σ" was evaluated as C Grade, "1.60 < σ ≤ 2.00" was evaluated as B Grade, "1.30 < σ ≤ 1.60" was evaluated as A Grade, and "σ ≤ 1.30" was evaluated as S Grade. B Grade or higher was determined to be acceptable, and C Grade was determined to be unacceptable. The results are as shown in Table 4.

### (3) Evaluation of Mechanical Strength

As an evaluation of mechanical strength, the Vickers hardness (HV) was measured by performing a Vickers hardness test (based on JIS Z 2244 (2009)). 10-point measurement (load: 200 gf, retention time: 10 seconds) was performed using a micro Vickers hardness tester (made by Matsuzawa Co., Ltd., model: AMT-X7FS). The average value of the 8 points excluding the maximum and minimum values of the 10-point Vickers hardness was defined as the Vickers hardness of the sample.

As a result of the Vickers hardness test, "HV < 300" was evaluated as C Grade, "300 ≤ HV < 350" was evaluated as B Grade, "350 ≤ HV < 400" was evaluated as A Grade, and "400 ≤ HV" was evaluated as S Grade. B Grade or higher was determined to be acceptable, and C Grade was determined to be unacceptable. The mechanical property evaluation results are also in Table 4.

As for metal materials in general, it is known that the hardness of the metal material increases as precipitated particles are finely dispersed and precipitated in parent phase crystal grains (the average inter-particle distance of precipitated particles decreases). From this, it can be said that the Vickers hardness by mechanical strength evaluation indirectly represents the degree of dispersion and precipitation of fine isothermal ω phase particles precipitated in β phase crystal grains of the parent phase.

### (4) Evaluation of Corrosion Resistance

As an evaluation of corrosion resistance, pitting potential measurement was performed by performing an anode polarization test of a metallic biomaterial (based on JIS T 0302 (2000)). The collected polarization test piece was attached to a crevice corrosion prevention electrode, a silver/silver chloride electrode was used as a reference electrode, and the potential V_{C100} (unit: mV (vs. Ag/AgCl)) with a current density of 100 mV/cm² was obtained from the polarization curve obtained by anode polarization in physiological saline (0.9% by mass NaCl solution).

As a result of the pitting potential measurement, "V_{C100} < 750 mV" was evaluated as C Grade, "750 mV ≤ V_{C100} < 1,000 mV" was evaluated as B Grade, "1,000 mV ≤ V_{C100} < 1,250 mV" was evaluated as A Grade, and "1,250 mV ≤ V_{C100}" was evaluated as S Grade. B Grade or higher was determined to be acceptable, and C Grade was determined to be unacceptable. The corrosion resistance evaluation results are also in Table 4.

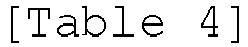

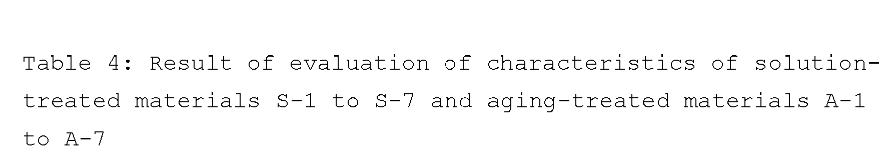

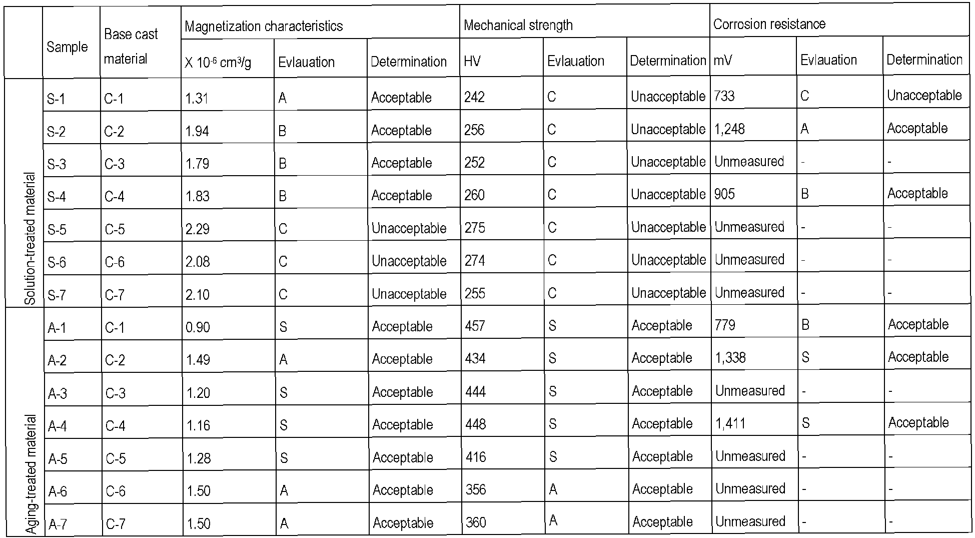

As shown in Table 4, comparing the solution-treated materials and aging-treated materials, the solution-treated materials S-1 to S-7 show unacceptable characteristics in any of the items of magnetization characteristics, mechanical strength, and corrosion resistance. On the other hand, the aging-treated materials A-1, A-2, and A-4 as compared with the solution-treated materials sharing the same base cast materials are at least equivalent to the solution-treated materials in every item. In addition, it is considered that A-3 and A-5 to A-7 also show an improvement in corrosion resistance from the results in magnetization characteristics and mechanical strength and the degree of improvement from the solution-treated material.

Considered together with the results in Fig. 5 and Table 3, these results are considered to be attributable to the aging-treated material having a microstructure in which fine isothermal ω phase particles are dispersed and precipitated in β phase crystal grains of the parent phase. More specifically, it is considered that the isothermal ω phase smaller in magnetic susceptibility than the β phase was precipitated in the aging-treated material and thus the magnetization characteristics were improved (mass magnetic susceptibility was lowered). In addition, it is considered that the isothermal ω phase particles were dispersed in β phase crystal grains of the parent phase and thus the corrosion resistance weakness attributable to the ω phase could be minimized. Further, it is considered that fine precipitated particles were dispersed and thus the Vickers hardness was improved.

The above embodiment and examples have been described so that the understanding of the present invention is facilitated, and the present invention is not limited to the specific configuration described above. For example, a part of the configuration of the embodiment can be replaced with configurations of the technological common sense of those skilled in the art and configurations of the technological common sense of those skilled in the art can be added to the configuration of the embodiment.

### Reference Signs List

10: molten metal
11: raw material alloy ingot
12: purified molten metal
20: cast material
30: solution-treated material
40: aging-treated material
50: machined material
60: plastically worked material
70: stent
80: spring guide wire
81: core

## Claims

1. A Zr-Nb-based alloy material comprising, as a chemical composition:
3% by mass or more and 18% by mass or less of Nb;
3% by mass or more and 12% by mass or less of Ti;
0.5% by mass or more and 6% by mass or less of Cr;
6% by mass or less of Cu;
5% by mass or less of Bi,
optionally 9% by mass or less of Ag, 6% by mass or less of Sn and 3% by mass or less of Al; and
a remainder consisting of Zr and unavoidable impurities, wherein the total impurity content is 0.5 mass % or less;
wherein isothermal ω phase particles are dispersed and precipitated in β phase crystal grains of a parent phase.

2. The Zr-Nb-based alloy material according to claim 1, wherein the isothermal ω phase particles have a chemical composition in which the Zr content is high and the Nb content is low as compared with the β phase crystal grains.

3. The Zr-Nb-based alloy material according to any one of claims 1 to 3, wherein the isothermal ω phase particles have an average particle size of 200 nm or less, the particle size being measured by microstructure observation with acceleration voltage 200 kV and magnification x 40,000 using a transmission electron microscope-energy dispersive X-ray analyzer.

4. The Zr-Nb-based alloy material according to any one of claims 1 to 3, wherein the Zr-Nb-based alloy material is
- 2.00 × 10⁻⁶ cm³/g or less in mass magnetic susceptibility β, the mass magnetic susceptibility being calculated from the relationship between an obtained magnetization, applied magnetic field, and sample mass and the magnetic magnetization being performed using a RIKEN Electronics Co., Ltd., vibration sample-type magnetometer BHV series as a magnetic material measuring device;
- 300 HV or more in Vickers hardness HV when the Vickers hardness (HV) is measured by performing a Vickers hardness test based on JIS Z 2244 (2009) by performing a 10-point measurement with load: 200 gf and retention time: 10 seconds using a micro Vickers hardness tester made by Matsuzawa Co., Ltd., model: AMT-X7FS and defining the average value of the 8 points excluding the maximum and minimum values of the 10-point Vickers as the Vickers hardness of the sample, and
- 750 mV or more in pitting potential V_{C100} when the pitting potential is measured by performing an anode polarization test of a metallic biomaterial based on JIS T 0302 (2000) by attaching a collected polarization test piece a crevice corrosion prevention electrode, using a silver/silver chloride electrode as a reference electrode, and obtaining the potential VC₁₀₀, unit: mV vs. Ag/ AgCl, with a current density of 100 mV/cm² from the polarization curve obtained by anode polarization in physiological saline 0.9% by mass NaCl solution.

5. A method for manufacturing the Zr-Nb-based alloy material according to any one of claims 1 to 4, the method comprising:
a raw material mixing and melting step of forming a molten metal by mixing and melting raw materials of the Zr-Nb-based alloy material;
a casting step of forming a cast material having the chemical composition by casting the molten metal;
a solution treatment step of preparing a solution-treated material by performing solution treatment at 950°C or more and 1,100°C or less on the cast material; and
an aging treatment step of obtaining an aging-treated material by performing aging treatment at 100°C or more and 450°C or less on the solution-treated material.

6. The method for manufacturing the Zr-Nb-based alloy material according to claim 5, further comprising a plastic working step of forming a plastically worked material by performing plastic working on the solution-treated material between the solution treatment step and the aging treatment step,
wherein the aging treatment step is performed on the plastically worked material.

7. The method for manufacturing the Zr-Nb-based alloy material according to claim 5, further comprising a plastic working step of forming a plastically worked material by performing plastic working on the cast material between the casting step and the solution treatment step,
wherein the solution treatment step is performed on the plastically worked material.

8. The method for manufacturing the Zr-Nb-based alloy material according to any one of claims 5 to 6,
wherein the raw material mixing and melting step includes
a raw material alloy ingot forming element step of forming a raw material alloy ingot by temporarily solidifying the molten metal after forming the molten metal by mixing and melting the raw materials; and
a remelting element step of preparing a purified molten metal by remelting the raw material alloy ingot.

9. A method for manufacturing the Zr-Nb-based alloy material according to any one of claims 1 to 4, the method comprising:
a powder preparation step of preparing additive manufacturing powder adjusted so as to have the chemical composition;
an additive manufacturing step of forming an additively manufactured product having a desired three-dimensional shape using the additive manufacturing powder;
a solution treatment step of preparing a solution-treated material by performing solution treatment at 950°C or more and 1,100°C or less on the additively manufactured product; and
an aging treatment step of obtaining an aging-treated material by performing aging treatment at 100°C or more and 450°C or less on the solution-treated material.

10. A Zr-Nb-based alloy product comprising a Zr-Nb-based alloy material, wherein the Zr-Nb-based alloy material is the Zr-Nb-based alloy material according to any one of claims 1 to 4 and the product is a stent or a spring guide wire.

## Patentansprüche

1. Zr-Nb-basiertes Legierungsmaterial umfassend, als chemische Zusammensetzung:
3 % Masse oder mehr und 18 % Masse oder weniger Nb;
3 % Masse oder mehr und 12 % Masse oder weniger Ti;
0,5 % Masse oder mehr und 6 % Masse oder weniger Cr;
6 % Masse oder weniger Cu;
5 % Masse oder weniger Bi;
optional 9 % Masse oder weniger Ag, 6 % Masse oder weniger Sn und 3 % Masse oder weniger Al;
und Rest bestehend aus Zr und unvermeidlichen Verunreinigungen, wobei der gesamte Gehalt an Verunreinigungen 0,5 % Masse oder weniger beträgt;
**dadurch gekennzeichnet, dass** isotherme ω-Phasen-Partikel in β-Phasen-Kristallkörnern einer Matrixphase dispergiert und präzipitiert sind.

2. Zr-Nb-basiertes Legierungsmaterial nach Anspruch 1,
**dadurch gekennzeichnet, dass** die isothermen ω-Phasen-Partikel eine chemische Zusammensetzung aufweisen, bei der der Zr-Gehalt im Vergleich zu den ß-Phasen-Kristallkörnern hoch und der Nb-Gehalt niedrig ist.

3. Zr-Nb-basiertes Legierungsmaterial nach einem der vorhergehenden Ansprüche 1 oder 2,
**dadurch gekennzeichnet, dass** die isothermen ω-Phasen-Partikel eine durchschnittliche Partikelgröße von 200 nm oder weniger haben, wobei die Partikelgröße durch Mikrostrukturobservation mit einer Beschleunigungsspannung von 200 kV und einer Vergrößerung von ×40 000 unter Verwendung eines Transmissionselektronenmikroskops mit energiedispersiver Röntgenanalyse gemessen wird.

4. Zr-Nb-basiertes Legierungsmaterial nach einem der vorhergehenden Ansprüche 1 bis 3,
wobei das Zr-Nb-basierte Legierungsmaterial
- eine Massenmagnetische Suszeptibilität × von 2,0 × 10⁻⁶ cm³/g oder weniger aufweist, wobei die Massenmagnetische Suszeptibilität aus dem Zusammenhang zwischen einer erhaltenen Magnetisierung, angelegtem Magnetfeld und der Probenmasse berechnet wird und die Magnetisierung unter Verwendung eines RIKEN Electronics Co., Ltd. Vibration Sample-Typ Magnetometers der BHV-Serie als magnetisches Materialmessgerät durchgeführt wird;
- 300 HV oder mehr in Vickershärte HV aufweist, wenn die Vickershärte (HV) durch Durchführung eines Vickershärtetests basierend auf JIS Z 2244 (2009) mit einer 10-Punkt-Messung bei einer Last von 200 gf und einer Haltezeit von 10 Sekunden unter Verwendung eines Mikro-Vickershärteprüfgeräts der Firma Matsuzawa Co., Ltd., Modell AMT-X7FS, gemessen wird und der Mittelwert von acht Messpunkten, abzüglich des Maximal- und Minimalwerts der 10-Punkt-Vickerswerte, als Vickershärte der Probe definiert wird; und
- ein Lochfraßpotential V_{C100} von 750 mV oder mehr aufweist, wenn das Lochfraßpotential durch Durchführung eines Anodenpolarisationstests an einem metallischen Biomaterial basierend auf JIS T 0302 (2000) gemessen wird, indem ein entnommenes Polarisationsteststück an eine Spaltkorrosionsschutzelektrode angeschlossen wird, eine Silber/Silberchlorid-Elektrode als Referenzelektrode verwendet wird und das Potential V_{C100} (Einheit: mV gegen Ag/AgCl) bei einer Stromdichte von 100 µA/cm² aus der durch Anodenpolarisation in physiologischer Kochsalzlösung (0,9 % Masse NaCl-Lösung) erhaltenen Polarisationskurve ermittelt wird.

5. Verfahren zur Herstellung eines Zr-Nb-basierten Legierungsmaterials nach einem der Ansprüche 1 bis 4,
umfassend:
- einen Rohmaterialmisch- und Schmelzschritt, bei dem durch Mischen und Schmelzen von Rohmaterialien des Zr-Nb-basierten Legierungsmaterials ein geschmolzenes Metall gebildet wird;
- einen Gießschritt, bei dem durch Gießen des geschmolzenen Metalls ein Gussmaterial mit der chemischen Zusammensetzung gebildet wird; einen Lösungsglühschritt, bei dem durch Durchführung einer Lösungsglühbehandlung bei 950 °C oder mehr und 1 100 °C oder weniger am Gussmaterial ein lösungsgeglühtes Material hergestellt wird; und
- einen Alterungsbehandlungsschritt, bei dem durch Durchführung einer Alterungsbehandlung bei 100 °C oder mehr und 450 °C oder weniger am lösungsgeglühten Material ein gealtertes Material erhalten wird.

6. Verfahren zur Herstellung eines Zr-Nb-basierten Legierungsmaterials nach Anspruch 5,
ferner umfassend einen zusätzlichen Umformschritt, bei dem durch Umformen eines lösungsgeglühten Materials zwischen dem Lösungsglühschritt und dem Alterungsbehandlungsschritt ein plastisch verformtes Material hergestellt wird, wobei der Alterungsbehandlungsschritt an dem plastisch verformten Material durchgeführt wird.

7. Verfahren zur Herstellung eines Zr-Nb-basierten Legierungsmaterials nach Anspruch 5,
ferner umfassend einen zusätzlichen Umformschritt, bei dem durch Umformen eines Gussmaterials zwischen dem Gießschritt und dem Lösungsglühschritt ein plastisch verformtes Material hergestellt wird, wobei der Lösungsglühschritt an dem plastisch verformten Material durchgeführt wird.

8. Verfahren zur Herstellung eines Zr-Nb-basierten Legierungsmaterials nach einem der Ansprüche 5 oder 6,
wobei der Rohmaterialmisch- und Schmelzschritt einen Rohmaterial-Legierungsbarren-Herstellungsschritt umfasst, bei dem durch temporäres Erstarren des geschmolzenen Metalls nach dessen Bildung durch Mischen und Schmelzen der Rohmaterialien ein Rohmaterial-Legierungsbarren erzeugt wird, und einen Umschmelzschritt, bei dem durch Umschmelzen des Rohmaterial-Legierungsbarrens ein gereinigtes geschmolzenes Metall hergestellt wird.

9. Verfahren zur Herstellung eines Zr-Nb-basierten Legierungsmaterials nach einem der Ansprüche 1 bis 4, wobei das Verfahren ferner umfasst:
einen Pulverbereitungsschritt, bei dem ein Additivherstellungspulver mit der chemischen Zusammensetzung vorbereitet wird; einen additiven Fertigungsschritt, bei dem unter Verwendung des Additivherstellungspulvers ein additiv gefertigtes Produkt mit der gewünschten dreidimensionalen Form gebildet wird; einen Lösungsglühschritt, bei dem durch Durchführung einer Lösungsglühbehandlung bei 950 °C oder mehr und 1 100 °C oder
weniger am additiv gefertigten Produkt ein lösungsgeglühtes Material hergestellt wird; und einen Alterungsbehandlungsschritt, bei dem durch Durchführung einer Alterungsbehandlung bei 100 °C oder mehr und 450 °C oder weniger am lösungsgeglühten Material ein gealtertes Material erhalten wird.

10. Zr-Nb-basiertes Legierungsprodukt, das ein ein Zr-Nb-basiertes Legierungsmaterial umfasst,
wobei das Zr-Nb-basierte Legierungsmaterial ein Zr-Nb-basiertes Legierungsmaterial nach einem der Ansprüche 1 bis 4 ist und das Produkt ein Stent oder ein Federführungsdrahtkern ist.

## Revendications

1. Matériau d'alliage à base de Zr-Nb comprenant, comme une composition chimique :
3% en masse ou plus et 18% en masse ou moins de Nb ;
3% en masse ou plus et 12% en masse ou moins de Ti ;
0,5% en masse ou plus et 6% en masse ou moins de Cr ;
6% en masse ou moins de Cu ;
5% en masse ou moins de Bi,
facultativement 9% en masse ou moins d'Ag, 6% en masse ou moins de Sn et 3% en masse ou moins d'Al ; et
un solde consistant en Zr et des impuretés inévitables, dans lequel la teneur totale en impuretés est 0,5% en masse ou moins ;
dans lequel des particules de phase isotherme ω sont dispersées et précipitées dans des grains cristallins de phase β d'une phase parent.

2. Matériau d'alliage à base de Zr-Nb selon la revendication 1, dans lequel les particules de phase isotherme ω ont une composition chimique dans laquelle la teneur en Zr est élevée et la teneur en Nb est basse par comparaison avec les grains cristallins de phase β.

3. Matériau d'alliage à base de Zr-Nb selon l'une quelconque des revendications 1 à 2, dans lequel les particules de phase isotherme ω ont une taille moyenne de particules de 200 nm ou moins, la taille de particules étant mesurée par observation de microstructure avec une tension d'accélération de 200 kV et un grossissement de x40 000 en utilisant un microscope électronique en transmission-un analyseur en dispersion d'énergie à rayons X.

4. Matériau d'alliage à base de Zr-Nb selon l'une quelconque des revendications 1 à 3, dans lequel le matériau d'alliage à base de Zr-Nb est
- 2,00 × 10⁻⁶ cm³/g ou moins en susceptibilité magnétique massique σ, la susceptibilité magnétique massique étant calculée à partir de la relation entre une magnétisation obtenue, un champ magnétique appliqué, et une masse d'échantillon et la magnétisation magnétique étant effectuée en utilisant un magnétomètre de type à échantillon vibrant série BHV de RIKEN Electronics Co., Ltd. comme un dispositif de mesure de matériau magnétique ;
- 300 HV ou plus en dureté Vickers HV lorsque la dureté Vickers (HV) est mesurée en effectuant un test de dureté Vickers sur la base de JIS Z 2244 (2009) en effectuant une mesure sur 10 points avec charge : 200 gf et temps de rétention : 10 secondes en utilisant un testeur de microdureté Vickers fabriqué par Matsuzawa Co., Ltd., modèle AMT-X7FS et en définissant la valeur moyenne des 8 points excluant les valeurs maximum et minimum des 10 points Vickers comme la dureté Vickers de l'échantillon, et
- 750 mV ou plus de potentiel de piqûration V_{c100} lorsque le potentiel de piqûration est mesuré en effectuant un test de polarisation anodique d'un biomatériau métallique sur la base de JIS T 0302 (2000) en fixant à une éprouvette de polarisation recueillie une électrode de prévention de corrosion fissurante, en utilisant une électrode argent/chlorure d'argent comme une électrode de référence, et en obtenant le potentiel VC₁₀₀, unité : mV versus Ag/AgCl, avec une densité de courant de 100 mV/cm² à partir de la courbe de polarisation obtenue par polarisation anodique dans une solution saline physiologique à 0,9% en masse de NaCl.

5. Procédé de fabrication du matériau d'alliage à base de Zr-Nb selon l'une quelconque des revendications 1 à 4, le procédé comprenant :
une étape de mélange et de fonte de matières premières pour former un métal fondu en mélangeant et en fondant des matières premières du matériau d'alliage à base de Zr-Nb ;
une étape de coulée pour former un matériau coulé ayant la composition chimique en coulant le métal fondu ;
une étape de traitement en solution pour préparer un matériau traité en solution en effectuant un traitement en solution à 950°C ou plus et 1 100°C ou moins sur le matériau coulé ; et
une étape de traitement de vieillissement pour obtenir un matériau traité par vieillissement en effectuant un traitement de vieillissement à 100°C ou plus et 450°C ou moins sur le matériau traité en solution.

6. Procédé de fabrication du matériau d'alliage à base de Zr-Nb selon la revendication 5, comprenant en outre une étape de travail plastique pour former un matériau travaillé plastiquement en effectuant un travail plastique sur le matériau traité en solution entre l'étape de traitement en solution et l'étape de traitement de vieillissement,
dans lequel l'étape de traitement de vieillissement est effectuée sur le matériau travaillé plastiquement.

7. Procédé de fabrication du matériau d'alliage à base de Zr-Nb selon la revendication 5, comprenant en outre une étape de travail plastique pour former un matériau travaillé plastiquement en effectuant un travail plastique sur le matériau coulé entre l'étape de coulée et l'étape de traitement en solution,
dans lequel l'étape de traitement en solution est effectuée sur le matériau travaillé plastiquement.

8. Procédé de fabrication du matériau d'alliage à base de Zr-Nb selon l'une quelconque des revendications 5 à 6,
dans lequel l'étape de mélange et de fonte de matières premières inclut
une étape élémentaire de formation de lingot d'alliage de matières premières pour former un lingot d'alliage de matières premières en solidifiant temporairement le métal fondu après la formation du métal fondu en mélangeant et en fondant les matières premières ; et
une étape élémentaire de refonte pour préparer un métal fondu purifié en refondant le lingot d'alliage de matières premières.

9. Procédé de fabrication du matériau d'alliage à base de Zr-Nb selon l'une quelconque des revendications 1 à 4, le procédé comprenant :
une étape de préparation de poudre pour préparer une poudre de fabrication additive ajustée de façon à avoir la composition chimique ;
une étape de fabrication additive pour former un produit fabriqué additivement ayant une forme tridimensionnelle désirée en utilisant la poudre de fabrication additive ;
une étape de traitement en solution pour préparer un matériau traité en solution en effectuant un traitement en solution à 950°C ou plus et 1 100°C ou moins sur le produit fabriqué additivement ; et
une étape de traitement de vieillissement pour obtenir un matériau traité par vieillissement en effectuant un traitement de vieillissement à 100°C ou plus et 450°C ou moins sur le matériau traité en solution.

10. Produit d'alliage à base de Zr-Nb comprenant un matériau d'alliage à base de Zr-Nb, dans lequel le matériau d'alliage à base de Zr-Nb est le matériau d'alliage à base de Zr-Nb selon l'une quelconque des revendications 1 à 4 et le produit est un stent ou un fil guide à ressort.
